# EUROPEAN PATENT APPLICATION

(11) **EP 4 811 386 A1**
(43) Date of publication of application: **23.09.2026**
(21) Application number: 25305378.9
(22) Date of filing: 19.03.2025
(51) Int. Cl.: G16H 30/40, G16H 50/20

(54) **FORMING A DATASET OF MEDICAL IMAGES**

(71) Applicant: Dassault Systèmes, 78140 Vélizy-Villacoublay (FR)
(72) Inventor: BALL, Arthur, Vélizy-Villacoublay (FR); BORNACHOT, Yohan, Vélizy-Villacoublay (FR)
(74) Representative: Bandpay & Greuter

(57) **Abstract**

The disclosure notably relates to a computer-implemented method for forming a dataset of medical images. The dataset is configured to be used for training a model. The trained model is thereby configured to output a prediction from an input medical image. The dataset-forming method comprises obtaining a first set of medical images. The first set consists of annotated medical images. The first set is unbalanced with respect to a meta-property relative to each medical image. The dataset-forming method further comprises obtaining a second set of medical images. The second set comprises unannotated medical images. The dataset-forming method further comprises outputting a third set of medical images. The third set comprises the first set and unannotated medical images of the second set. The third set is balanced with respect to the meta-property. The dataset-forming method is an improved solution for forming a dataset of medical images.

## Description

### TECHNICAL FIELD

The disclosure relates to the field of computer programs and systems, and more specifically to a methods, systems, programs, and a model related to forming a dataset of medical images.

### BACKGROUND

The performance of a machine-learning model in performing a particular task, such as multi-class classification, regression, detection, and/or instance segmentation, highly depends on the dataset it is trained on. To optimize performance, the dataset should be representative of the reality. Moreover, the dataset should also comprise enough data so that the model can learn the complex relations that lie between the inputs and desired outputs during training.

Within this context, there is still a need for an improved method for forming a dataset of medical images configured to be used for machine-learning.

### SUMMARY

It is therefore provided a computer-implemented method for forming a dataset of medical images. The dataset is configured to be used for training a model. The trained model is thereby configured to output a prediction from an input medical image. The method may be referred to as the "dataset-forming method" in the present disclosure.

The dataset-forming method comprises obtaining a first set of medical images. The first set consists of annotated medical images. The first set is unbalanced with respect to a meta-property relative to each medical image. The dataset-forming method further comprises obtaining a second set of medical images. The second set comprises unannotated medical images. The dataset-forming method further comprises outputting a third set of medical images. The third set comprises the first set and unannotated medical images of the second set. The third set is balanced with respect to the meta-property.

The method may comprise one or more of the following:
- the prediction comprises a segmentation of the input medical image;
- each annotated medical image of the first set has at least one annotation that represents a lesion, and the segmentation comprises a lesion segmentation, the lesion optionally being a tumor;
- the unannotated medical images of the second set included in the third set comprise medical images where a lesion is represented;
- each medical image is a slice image of a respective patient, optionally a slice of a Computed Tomography scan (CT scan) of the respective patient;
- the meta-property comprises a representation of a normalized height of the slice image;
- the representation of the normalized height is a result of a pretrained model configured to take a medical image as input, and to predict a value for the normalized height;
- the third set of medical images is partitioned into subsets of medical images, each subset corresponding to a respective atom of a partitioning of the range of the meta-property, each atom of the partitioning having a same size; and/or
- at least one of the subsets contains exclusively annotated medical images of the first subset.

It is further provided a computer-implemented method for machine-learning a model. The model being thereby configured to output a prediction from an input medical image. The method may be referred to as the "machine-learning method" in the present disclosure.

The machine-learning method comprises obtaining a dataset formed according to the dataset-forming method. The dataset is the outputted third set. The machine-learning method further comprises training the model on the dataset.

It is further provided a model obtainable according to the machine-learning method, that is, a computer-implemented model having values of weights and parameters set by the machine-learning method. The provided model may for example have been learnt directly by the machine-learning method, with its weights and parameters having been fixed by the training step of the machine-learning method.

It is further provided a computer-implemented method for using a model machine-learnt according to the machine-learning method. The method may be referred to as the "method of use" in the present disclosure.

The method of use comprises obtaining a medical image. The method of use further comprises applying the machine-learnt model to the medical image.

It is further provided a computer program comprising instructions for performing the dataset-forming method, the machine-learning method, and/or the method of use.

It is further provided a device comprising a data storage medium having recorded thereon the computer program and/or the model.

The device may form or serve as a non-transitory computer-readable medium, for example on a SaaS (Software as a service) or other server, or a cloud based platform, or the like. The device may alternatively comprise a processor coupled to the data storage medium. The device may thus form a computer system as a whole or in part (e.g., the device is a subsystem of the overall system). The system may further comprise a graphical user interface coupled to the processor.

### BRIEF DESCRIPTION OF THE DRAWINGS

Non-limiting examples will now be described in reference to the accompanying drawings, where:
- FIG. 1 shows a flowchart of an example of the dataset-forming method;
- FIGs. 2-12 illustrate example implementations of the method; and
- FIG. 13 shows an example of the system.

### DETAILED DESCRIPTION

It is proposed a computer-implemented method for forming a dataset of medical images. The dataset is configured to be used for training a model. The trained model (i.e., the model trained based on the dataset) is thereby configured to output a prediction from an input medical image. The method may be referred to as the "dataset-forming method" in the present disclosure.

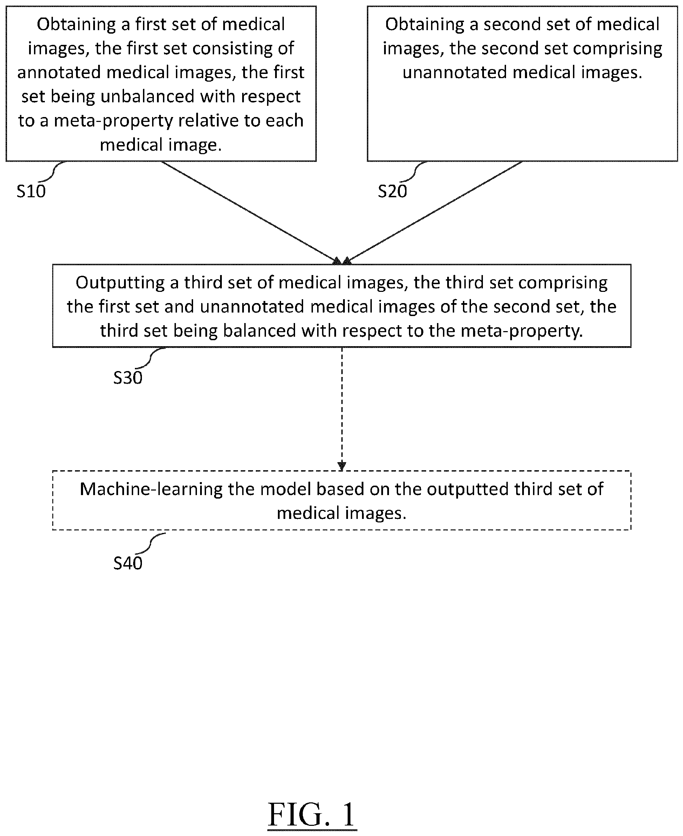

FIG. 1 shows a flowchart of an example of the dataset-forming method, comprising two independent steps S10 and S20 that may be performed in any order in the dataset-forming method (e.g., in parallel). The dataset-forming method further comprises a step S30 requiring as input the output of both S10 and S20. The method according to the example further comprises a step S40 based on the output of S30. The step S40 is optional in the dataset-forming method and will be described further below in the present disclosure.

With reference to the flowchart of FIG. 1, the dataset-forming method comprises obtaining S10 a first set of medical images. The first set consists of annotated medical images. The first set is unbalanced with respect to a meta-property. The meta-property is relative to each medical image. The dataset-forming method further comprises obtaining S20 a second set of medical images. The second set comprises unannotated medical images. The dataset-forming method further comprises outputting S30 a third set of medical images. The third set comprises the first set and unannotated medical images of the second set. The third set is balanced with respect to the meta-property.

The quality of trained machine learning models highly depends on the quality of the training dataset used during the training of the model. Training a model with unbalanced training datasets, where one class significantly outweighs other classes in terms of sample size, can lead to biased trained model incapable of doing accurate predictions. Models that are trained on unbalanced datasets may prioritize the majority class, resulting in poor generalization to the minority classes. This can be particularly problematic in classification tasks where each class holds equal importance. Balanced datasets ensure that the model learns from sufficient examples of each class, enabling it to make fair and accurate predictions across all classes. Moreover, a balanced dataset fosters better model understanding and robustness, enhancing its capability to handle real-world scenarios with varying class distributions.

The dataset-forming method is an improved solution as it allows to form balanced datasets of medical images. The dataset-forming method forms a balanced dataset comprising all the annotated images (e.g., the images of the first set) and at least part of the unannotated images (e.g., at least some of the images of the second set). Therefore, the dataset-forming method adds images to and/or balances the first set of medical images, using images from the second set of medical images.

Prior art methods comprised under-sampling and over-sampling, or a combination of both processes, as disclosed in A. Stando, M. Cavus, P. Biecek (2023). The Effect Of Balancing Methods On Model Behavior In Imbalanced Classification Problems. arXiv:2307.00157. However, the method of over-sampling can also lead to worse model performance, as disclosed in R. Channing Moore, Daniel P. W. Ellis, Eduardo Fonseca, Shawn Hershey, Aren Jansen, Manoj Plakal (2023). Dataset Balancing Can Hurt Model Performance. arXiv:2307.00079v1.

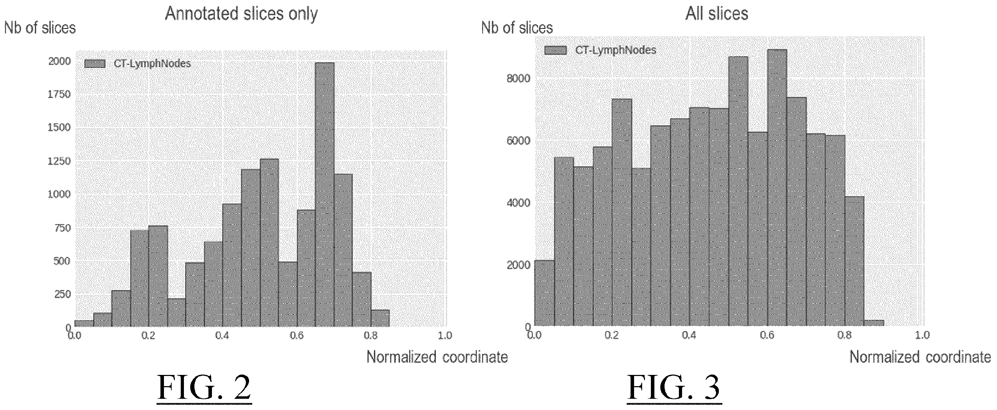

Moreover, medical images datasets often have less data samples than other datasets and have less ground-truth annotations. Indeed, such annotations may only be done by health specialists. If the method of under-sampling is applied to such dataset, the amount of data may not be sufficient for training a model on the desired task efficiently. In contrast, unannotated images may be abundant (as discussed in more detail later with reference to FIGs. 2 and 3). In these cases, the set of annotated images (the "first set") may be significantly smaller than the set of unannotated images (the "second set").

For example, the dataset-forming method may comprise or may be performed after a process that comprises obtaining a dataset of medical images consisting of slices of CT scans. A CT scan consists of several 2D slices of a patient and may be used to see the progression of a disease (e.g., cancer). The dataset-forming method or the process may further comprise annotating (e.g., by a health specialist) a slice of each CT scan comprising at least one lesion. The annotated slices may be a slice of a respective CT scan wherein the lesion is most visible. The obtaining S10 may comprise selecting the (or part of the) annotated images of the obtained dataset and the obtaining S20 may comprise selecting the (or part of the) unannotated images of the obtained dataset. Clearly, in such example, the second set may be much bigger than the first. The dataset-forming method fully leverages this unbalance and optimally form a balanced dataset comprising all of the annotated images.

The dataset-forming method enables the formation (at S30) of a dataset of medical images (i.e., the third set) that is balanced with respect to the meta-property. Although the meta-property may not be directly related to the output of the model to be trained, a dataset that is balanced with respect to the meta-property can lead to a more complete and robust data representation within the model, thereby enhancing the performance for the prediction output of the model. Moreover, a balanced dataset can also lead to a better model generalization and allow obtaining a more robust internal representation of the dataset.

The dataset-forming method may comprise machine-learning S40 the model based on the outputted third set of medical images. Alternatively or additionally, the outputted third set may be stored, such as on volatile memory or on cloud memory, and/or sent to a distant system, so as to be later used for such a machine-learning. The model is in all cases configured to output a prediction from an input medical image.

It is indeed provided a computer-implemented method for machine-learning a model. The method may be referred to as the "machine-learning method" in the present disclosure.

The machine-learning method comprises obtaining a dataset formed according to the dataset-forming method. The dataset is the outputted third set. The machine-learning method further comprises training the model on the dataset.

As detailed above, training a model based on a balanced dataset ensures that the model (after the training) does not exhibit biases with respect to the meta-property (e.g., does not overly mis-predict the input medical image corresponding to underrepresented values of the meta-property).

It is further provided a computer-implemented method for use of a model machine-learnt according to the machine-learning method. The method may be referred to as the "method of use" in the present disclosure.

The method of use comprises obtaining a medical image. The method of use further comprises applying the machine-learnt model to the medical image.

The method of use, the dataset-forming method, and/or the machine-learning method may be combined in any possible way. For example, the dataset-forming method may comprise the learning method (e.g., S40). The dataset-forming method may further comprise using the model trained with the learning method (e.g., trained at S40) to obtain a prediction (e.g., comprising obtaining an input medical image and/or providing the input medical image to the trained model).

A dataset comprising medical images may be heavily unbalanced as some diseases may occur with a much higher frequency than other diseases. In examples wherein the annotations correspond to tumors, annotations corresponding to, e.g., lung tumor, may be more frequent than annotations corresponding to rarer tumors. Therefore, a dataset comprising medical images may be highly unbalanced and training a model using such unbalanced dataset may negatively impact the ability of the trained model to detect the rarer diseases. The dataset-forming method provides a dataset (the outputted third set of images) that is balanced (e.g., with respect to the kind of disease, or the location in the body). Using such balanced dataset, increases the reliability of the model trained in the machine-learning method and used in the method of use.

The dataset-forming method may comprise forming a dataset of medical images (i.e., the outputted third set of medical images), the dataset being configured to be used for training a model used in a digital-twin solution. That is, the method of use may be part of a digital-twin software solution and the dataset forming method may also be part of the digital-twin solution (e.g., comprising the trained model) itself. The digital (or virtual) twin of a patient may be a virtual representation (i.e., "digital twin") of the patient (i.e., "physical twin"). The digital twin may rely on model-based simulations of complex physics phenomena that may be parametrized to fit the patient's specificities. The digital twin may serve as a tool for medical applications, such as preoperative planning, procedural simulation, educational purposes, or the development and testing of medical devices. The digital twin may comprise several detection, prediction, and/or measurement tools that may be calibrated with a training dataset.

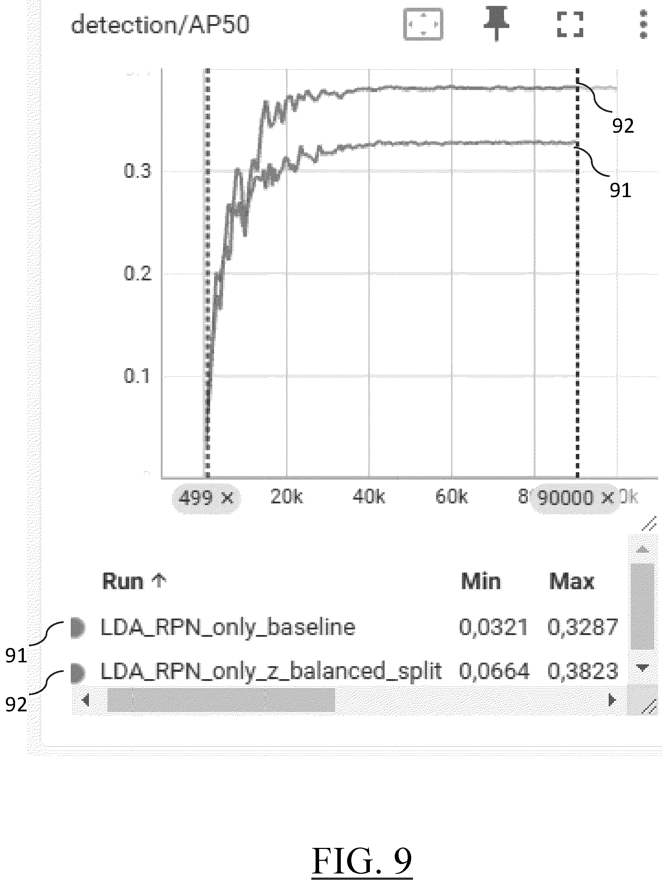

The dataset-forming method, the machine-learning method, and the method of use were implemented and the results of three different implementations are discussed herein below in reference to FIGs. 9-12. The results of the implementations clearly show the efficacy of the dataset-forming method. In these implementations, the models trained using a balanced dataset (i.e., the outputted third set of medical images) performed better than similar models (that is, models based on the same architecture) trained on an unbalanced dataset (i.e., the first set of medical images). Moreover, the models trained using the third set performed better than similar models trained exclusively based on medical images corresponding to a specific meta-property value or range. That is, models trained on the larger dataset (i.e., the third set of medical images) perform better than models trained on a restricted dataset (based on medical images corresponding to a specific meta-property value or range) even on the medical images corresponding to the specific meta-property value or range. Therefore, the dataset-forming method and the machine-learning method allow to train models able to output more reliable predictions both for solving specific predictions for medical images corresponding to specific values/ranges of the meta-property, and for predictions of medical images without an a priori knowledge of the meta-property.

Any model herein (e.g., the model of the machine-learning method and/or of S40) may be specified by its architecture, parameters, and hyperparameters. In examples, the model may be a neural network. The neural network may comprise different modules. In examples, the neural network may comprise, or be based on, a convolutional neural network. In other examples, the neural network may comprise, or be based on, a MaskR-CNN. The architecture of the model (e.g., of the neural network) may consist of layers, starting with the input layer of which neuron count may be equal to the dimensionality of the input data. The input layer may be followed by several hidden layers with a given number of neurons and activation functions. These layers and neurons define the model's depth and width. The model may comprise activation functions, e.g., in between layers, which may introduce nonlinearity into the model. The interconnections between these layers define the topology of the model. The parameters of the model are the learnable weights and biases, which are determined/configured/modified in the training process. In contrast, the hyperparameters are pre-defined values/settings that are not learned from the training data. These encompasses the number of hidden layers, neurons per layer and much more.

The machine-learning method comprises obtaining a training dataset of training examples (i.e., the third set of medical images outputted by the dataset-forming method). The training dataset is to be used for machine-learning/training the model. Some of the training examples may each further comprise a corresponding annotation (e.g., the medical images originally from the first set of medical images). Such an annotation is a ground truth of a phenomenon to be predicted. Other training examples may comprise no annotation (e.g., some of the medical images originally from the second set of medical images). The absence of any annotation on an image may be interpreted as meaning that the phenomenon does not occur for the specific example of said image, in other words there is nothing to predict from the image (e.g., no lesion/disease to predict/segment).

In implementations, the machine-learning method and/or S40 may comprise data preparation and/or data pre-processing prior to the training. Indeed, different medical images may have different standards (e.g., color level), and the trained model may benefit from a pre-processing that brings to similar level the different standards.

The machine-learning method and/or S40 comprise training a model based on the third set of medical images. The training (or equivalently machine-learning) of the model comprises providing the medical images from the third set as input to the model. Each of the medical images is processed through the model by multiplying values of weights (e.g., to values of pixels) applying activation functions, and combining results across layers. The training may then comprise comparing the output of the neural network against the annotation (if present) of the medical image. The comparison may comprise/compute an error. The comparison may comprise outputting an error based on a loss function. The loss function may quantify the difference between the prediction and the expected outcome. The error (e.g., the outputted error) may then be used to backpropagate through the network, leading to adjustments in the weights to minimize the error. Various optimization solvers, each having their own set of hyperparameters, may be employed during the training process to efficiently converge toward an optimal set of parameters. Hence, as is known *per se* from the field of machine-learning, the training of a neural network (e.g., the neural network of the learning method) comprises tuning the weights, biases, and other parameters of the network so that the processing of an input accurately predicts the occurrence of the clinical event.

The medical images (e.g., the medical images of the first set, of the second set, and/or the obtained medical image of the method of use) are each a visual representation of a respective part of the body of a respective patient. Each of the medical images may consist of a grid of points (e.g., grid of pixels in case of a 2D medical image, or voxels in case of a 3D medical image, each pixel or voxel may be associated with a value, such as an intensity value, such as grayscale value). The part of the body of respective patients may comprise any patient's tissue or anatomical structure, such as one or more organs, one or more bones, one or more muscles, one or more blood vessels, one or more nerves, cartilage, and/or other anatomical structures. One or more of the medical images may comprise one or more lesions or one or more abnormal growths. Medical images may be used for diagnostic, therapeutic, or research purposes and can encompass various formats, resolutions, and dimensions, depending on the imaging modality and application context.

The medical images (e.g., the medical images of the first set, of the second set, and/or the obtained medical image of the method of use), may each be an image (e.g., real images being obtained) obtained from any one of the following medical-imaging modalities: Autorefraction, Angioscopy, Bone Densitometry (US), Biomagnetic Imaging, Bone Densitometry (X-Ray), Color Flow Doppler, Cinefluoroscopy, Colposcopy, Computed Radiography, Cystoscopy, Computed Tomography (CT scan), Duplex Doppler, Digital Fluoroscopy, Diaphanography, Digital Microscopy, Digital Subtraction Angiography, Digital Radiography, Echocardiography, Electrocardiography, Cardiac Electrophysiology, Endoscopy, Fluorescein angiography, Fiducials, Fundoscopy, General Microscopy, Hard Copy, Hemodynamic Waveform, Intra-Oral Radiography, Intraocular Lens Data, Intravascular Optical Coherence Tomography, Intravascular Ultrasound, Keratometry, Lensometry, Laparoscopy, Laser Surface Scan, Magnetic Resonance Angiography, Mammography, Magnetic Resonance, MR T1 weighted, MR T2 weighted, MR Proton density weighted, MR Steady-state-free precession, MR Effective T2, MR Susceptibility-weighted, MR Short-tau inversion recovery, MR Fluid-attenuated inversion recovery, MR Double inversion recovery, MR Conventional diffusion weighted, MR Apparent diffusion coefficient, MR Diffusion tensor, MR Dynamic susceptibility contrast, MR 25 Arterial spin contrast, MR Dynamic contrast enhanced, MR Blood-oxygen-level dependent imaging, MR Time-of-flight, MR Phase contrast, Magnetic Resonance Spectroscopy, Nuclear Medicine, Ophthalmic Axial Measurements, Optical Coherence Tomography (non-Ophthalmic), Ophthalmic Photography, Ophthalmic Mapping, Ophthalmic Refraction, Ophthalmic Tomography, Ophthalmic Visual Field, Optical Surface Scan, Other, Positron Emission Tomography (PET), Panoramic X-Ray, Respiratory Waveform, Radio Fluoroscopy, Radiographic Imaging (conventional film/screen), Radiotherapy Dose, Radiotherapy Image, Radiotherapy Plan, Radiotherapy Treatment Record, Radiotherapy Structure Set, Segmentation, Slide Microscopy, Stereometric Relationship, Single-Photon Emission Computed Tomography (SPECT), Automated Slide Stainer, Thermography, Ultrasound, A-mode US, B-mode US, M-mode US, Visual Acuity, Videofluorography, X-Ray Angiography, and External-Camera Photography.

In examples, all of the medical images of the first and/or of the second set of medical images may be obtained with a same medical-imaging modality (e.g., all with CT scans). All of the medical images may represent the same part of bodies of respective patients or may represent a part being one among a predetermined list of parts of bodies. For example, all the medical images may be slices of CT scans, each slice representing a section of a body, the section being comprised between two anatomical reference points in a respective patient (e.g., each slice being a slice of a respective torso, or being a slice situated between a respective knee and a respective neck).

It is understood that the medical image obtained for the method of use (to be used as input for the model) may be of the same kind of image as the images comprised in the training dataset, that is, of the third set of medical images, and hence of the first and second set of medical images. For example, if the third set of medical images consists of slice images, (e.g., of a CT scan) the medical image obtained for the method of use may also be a slice image.

The medical images of the first set are annotated, meaning that each medical image of the first set includes supplementary information called "annotations" (such as labels, segmentation masks, and/or markers). The supplementary annotations may specify (e.g., by highlight, any form of description, or any form of identification) specific regions, features, and/or abnormalities present in the image. In examples, the supplementary annotations may delimitate a part of the image (e.g., corresponding to a lesion). Additionally or alternatively, the supplementary annotations may describe a part of the image. For example, the annotations may describe an organ represented in the image or a type or disease that may be visualized (e.g., that may have also been delimitated, as explained above) in the image. The supplementary annotations may have been manually added by health professionals.

A value of the meta-property is assigned to each medical image. That is, to each medical image corresponds a value of the meta-property. The value of the meta-property is an element of the range of the meta-property. The range may be a finite set or a numerical range (e.g., an interval, such as [0,1]). The meta-property may exhibit a predictive power (e.g., correlate) with the prediction generated by the model to be trained. However, the meta-property may not be directly related to said prediction (e.g., may only partially correlate). Moreover, the meta-property may pertain to the entire medical image rather than to a portion thereof, and it may be relevant both for the prediction (e.g., by being utilized during model training) and for a health specialist's examination of the image.

For example, a meta-property may include, but is not limited to, an indicator of a disease type (e.g., type of cancer), that may, e.g., be identified in an annotation of the medical image, a manufacturer of a scanner (e.g., used for a CT scan) used to obtain a corresponding medical image, phase contrast, or a positional information of a corresponding medical image (e.g., a height information of a slice image) within the body.

Optional features of the proposed solution are now discussed.

The value of the meta-property of associated medical images may or may not be used during the training of the model and/or may or may not be used by the trained model during inference. As the meta-property may correlate with the prediction, it may be beneficial to use the meta-property during training and/or inference.

Each medical image (e.g., comprised in the first set, the second set, and/or the obtained medical image of the method of use) may be a slice image of a respective patient. Each slice image may be substantially cross-sectional/transverse to the height of the patient (e.g., parallel to the ground if the patient is standing, transverse to a spine). Each of the medical images may, therefore, comprise an illustration of an internal anatomy of a patient, the illustration may be at a given height and transversal to the spine.

One or more (e.g., each) of the annotated medicals of the first set of medical images may each comprise one or more annotations each representing a respective lesion. Each of the represented lesions may be a portion of tissue of the body of the patient subject to a physiological condition. In examples, lesions may indicate various conditions, such as inflammations, or injuries. In examples, a lesion may be a tumor. The tissue may correspond to an organ (such as the liver, lungs, heart, or bones).

One or more (e.g., each) of the annotated medical images of the first set of medical images may comprise annotations representing an identification (e.g., segmentation mask) of a region comprising a lesion. Additionally or alternatively, one or more (e.g., each) of the annotated medical images of the first set of medical images may comprise annotations providing specifications about the lesion (e.g., a size, a kind of lesion). In examples, the identification and/or segmentation mask may comprise a bounding box enclosing the lesion.

Segmentation (or instance segmentation) is a process of automatically calculating a 2D or 3D segmentation mask of a given object (e.g., a lesion) in a medical image. In other words, the process of segmentation classifies the pixels (of the medical image) as belonging to the object (e.g., lesion) or not. The segmentation mask may be dense (e.g., not have any hole, e.g., simply connected) and connected (e.g., consisting of a single shape). In some examples, the segmentation mask may have a geometrical shape (e.g., a square) substantially containing (e.g., containing more than 90%) the object to be segmented (e.g., the lesion). In other examples, the segmentation mask may have a shape exactly identifying the object to be segmented (e.g., the lesion). The segmentation mask may be identified by a contour or boundary, signifying that anything enclosed in such contour/boundary is part of the object (e.g., part of the lesion). Such segmentation masks present a lightweight structure (by only comprising a boundary information and not the entire region information).

One or more (e.g., each) of the annotated medical images of the first set of medical images may comprise annotations comprising the information of a presence of a lesion, without the specification of a region comprising the lesion (e.g., the image may be annotated declaring a presence of a lesion, such as a tumor, without a segmentation mask of the image detecting said lesion).

The unannotated medical images of the second set of medical images may be medical images representing healthy tissues and/or organs, that is, without lesions, e.g., without tumors. Therefore, in examples, the model trained on the third dataset may use such unannotated images as examples of images without anything to segment/to detect.

The medical images (e.g., the medical images of the first set, of the second set, and/or the obtained medical image of the method of use) may be slices of a Computed Tomography scan (CT scan) of respective patients. CT scan is a medical imaging technique that is used to obtain detailed images of the body. CT scans produce images by sending X-rays through the human body. CT scans produce 3D images consisting of one or more slices (that are 2D images). The slices may be parallel to the horizontal plane (i.e., perpendicular to the height of the person). The slices are at varying distance from each other, the distance being, e.g., up to the order of millimeters. Therefore, a single CT scan may comprise a large number of 2D slices, e.g., a slice every few (e.g., 1 or 5) millimeters of a large zone of a body (e.g., entire body, or a thorax) of a patient.

The prediction of the model may comprise a segmentation of the input medical image. For example, the annotations of the medical images of the first set may be identifications of respective regions (e.g., segmentation masks), and the model trained (or to be trained) using the third set of medical images may be trained to segment the medical images as in the training dataset. Additionally or alternatively, each annotated medical image of the first set may have at least one annotation that represents a lesion, and the segmentation may comprise a lesion segmentation. Accordingly, the model trained using the third set of medical images may be trained to detect, classify, and/or segment a lesion. The detection task may be performed directly (e.g., without segmentation), or it may be derived from the segmentation task (e.g., by counting connected components). Optionally, the lesion (for at least one - e.g., each - annotation) may be a tumor.

The unannotated medical images of the second set included in the third set may comprise medical images where a lesion is represented (or alternatively no such unannotated medical image at all). That is, some of the medical images of the second set (that are selected to be part of the third set) may represent a lesion that was not annotated. The presence of such images in the third set of images does not affect (at least significantly) the performance of a model trained based on the third dataset. That is, the presence of such images does not increase (at least significantly) the number of errors of a model trained based on the third dataset. Indeed, the annotations (e.g., segmentation masks) typically concern a small part of the medical image, and the omission of some of the annotations does not perturb the training process. Thus, a user of the methods of the present disclosure may not have to check all of the unannotated images, e.g., to be sure that no lesion is represented.

The presence of unannotated medical images of the second set representing a lesion may be due to different factors. For example, a health specialist may not have seen a lesion while reviewing a medical image. Additionally or alternatively, the medical images may be slices of a CT scan and a health specialist may have only annotated one of the slices comprising the lesion (e.g., a slice wherein the lesion has a largest diameter). However, the lesion may be still visible in neighboring slices and not be annotated as such. Such unannotated images may be included in the second set.

In examples, the model may be based on (e.g., comprise) a convolutional neural network (CNN), a transformer, or an hybrid architecture comprising both a CNN and a transformer. The model may for example comprise a convolutional neural network that outputs a segmentation of the image provided as input.

As is known in the art, a CNN is a neural network architecture based on convolution layers and is used for feature extraction. A CNN may be trained exclusively based on a dataset of annotated images (that is, unannotated images are not necessary for the training). For example, the annotations may be segmentation masks corresponding to lesions that account for a small proportion of each of the annotated medical images. Therefore, a CNN could be trained exclusively based on the first set of medical images, however doing so, as explained above, would compromise the reliability of the prediction of the CNN, especially regarding medical images corresponding to the underrepresented values of the meta-property. Contrary to this approach, the machine-learning method comprises training a model comprising such a CNN based on a dataset that includes both annotated images and unannotated images. The dataset is balanced (obtained with the dataset-forming method). Thus, the model comprising such a CNN trained according to the machine-learning method reaches higher performances (e.g., accuracy of the model) compared to models only trained based on the first set of medical images.

As is known in the art, a transformer is a type of deep neural network architecture, which is capable of perceiving relationships among elements of the input. The relationships are obtained using a mechanism called "self-attention". Model comprising transformers may, thus, learn the relevance of each element of the input with respect to the others and weigh the contextual information appropriately.

The method of use may comprise, after the applying, displaying the output of the machine-learnt model. The displaying may be on a device, e.g., on a screen of a computer system.

The method of use may enable a health specialist to obtain predictions based on a set of medical images. The predictions may each comprise a segmentation of the input medical image, the segmentation optionally representing a lesion such as a tumor. Therefore, the health specialist may quickly (e.g., immediately after performing the method) obtain as output whether a tumor is present. In examples, the output may further comprise a segmentation of said tumor and, e.g., a size of the tumor. Therefore, the method of use may be used to measure a size of a tumor. This may be particularly advantageous when processing a large number of medical images. For example, an implementation of the method of use may comprise obtaining several medical images (e.g., a CT scan of a patient, or of several patients), applying the machine-learnt model to each of the medical images, and outputting which of the medical images comprises a lesion. Thanks to the proposed solution, the health specialist does not have to check each image nor guess which zones of the body of the patient may comprise a lesion. The output of the method of use may trigger a medical action (e.g., by a health specialist obtaining and/or seeing the result) such as diagnosis, further medical tests, and/or medical treatment (e.g., new treatment or treatment adaptation), based on the result of the comparison.

The method of use may additionally or alternatively comprise (e.g., automatically) processing the result of applying the machine-learnt model to the medical image so as to determine a diagnosis, and/or a new treatment or a manner to adjust an existing treatment of a patient, or any other medical action to be performed with respect to the patient.

The obtaining S10 and/or S20 may comprise retrieving (e.g., from local or remote memory, e.g., from a non-volatile memory) or receiving (e.g., from a remote system, e.g., over a network) the respective sets of medical images. Additionally or alternatively, the obtaining S10 and/or S20 may comprise obtaining a subset of medical images of the first and/or second set of medical images with a medical measuring device, e.g., by a health specialist. The obtaining S10 may also comprise annotating one or more medical images (e.g., segmenting the images, or identifying a region representing a lesion such as a tumor), the annotating may be effectuated by a health specialist. The obtaining S10 and S20 may also comprise obtaining a dataset of medical images, defining the first set of medical images as the subset of the dataset consisting of annotated images, and defining the second set of medical images as the complement of the first set.

The outputting S30 of a third set of medical images may comprise storing the outputted third set of medical images on a non-volatile memory and/or sending the third set of medical images to a remote server, e.g., over a network. The outputting S30 may comprise determining the third set of medical images. The determining may be effectuated with operations performed on a volatile memory (e.g., RAM).

The first set of medical images obtained at S10 is unbalanced with respect to a meta-property relative to each medical image, whereas the outputted (at S30) third set of medical images is balanced with respect to said meta-property.

A set of medical images (e.g., the third set, but not the first set) is said to be "balanced" with respect to a meta-property if the number of medical images comprised in the set corresponding to the non-outlier (or, equivalently, non-extremal) values of the meta-property is substantially the same. By "substantially the same" it is meant that the ratio between the numbers is close to 1, e.g., between 0.9 and 1.1, or equal to 1. That is, a set of medical images is balanced with respect to a meta-property if, for each given non-outlier value (or given range of non-outlier values) of the meta-property, the number of medical images comprised in the subset of medical images of which value of the meta-property is the given non-outlier value (or within the given range of non-outlier values) does not substantially depend on the given non-outlier value (or given range of non-outlier values). According to the above definition, by "substantially depends" it is meant that the ratio between the numbers is not close to 1 (e.g., not between 0.9 and 1.1, or not 1).

A set of medical images not satisfying such condition is said to be unbalanced (e.g., the first set). Therefore, a set of medical images is unbalanced if the number of medical images comprised in the subset of medical images of which value of the meta-property is a given non-outlier value (or within a given range of non-outlier values) substantially depends on the given non-outlier value (or on the given range of non-outlier values).

Outlier (or extremal) values and/or ranges of values of the meta-property may correspond to values and/or ranges of values that are underrepresented in both the first and the second set of medical images (e.g., extremely rare, or not pertinent medical images). The meta-property may have no outlier values or ranges of values. Alternatively, in examples where the meta-property has values in the interval [0,1], the outlier/extremal values may be the ranges of values near "0" or near "1", e.g., [0,0.1] and/or [0.9,1]. In a balanced set of medical images, the number of medical images corresponding to outlier values and/or ranges of values may be smaller (e.g., substantially smaller, e.g., with a ratio smaller than 0.9) than the others. The subset of medical images corresponding to the values and/or ranges of values of the meta-property that are outliers may be referred by outlier subsets of medical images.

The dataset-forming method may comprise determining the third set of medical images. The determining may comprise adding to the first set of medical images, medical images of the second set. The added medical images of the second set may correspond to values of the meta-property that are underrepresented in the first set of medical images, so that the determined set of medical images is balanced. Therefore, the determining of the third set of medical images may comprise balancing the first set of medical images with medical images of the second set of medical images.

FIGs. 2 and 3 illustrate vertical bar charts, where the horizontal axis indicates the meta-property to be balanced and the vertical axis indicates a number of images for the given meta-property range in the respective dataset. In specific, FIG. 2 illustrates a vertical bar chart of an example of a first set consisting of annotated medical images and FIG. 3 illustrates a vertical bar chart of an example of a second set. As can be seen, both sets are unbalanced, the number of medical images corresponding to the ranges of the meta-property given in the horizontal axis substantially depends on the ranges of the meta-property.

A balanced set of medical images (e.g., the third set of medical images of the dataset-forming method, but not the first set) may be partitioned into subsets of medical images, each subset corresponding to a respective atom of a partitioning of the range of the meta-property. Each atom of the partitioning may have a same size. Some of the atoms (e.g., extremal atoms) of the partitioning may be outliers atoms, or alternatively, none of the atoms of the partitioning may be an outlier atom. All the subsets of medical images corresponding to non-outlier atoms may have substantially the same cardinality (e.g., the ratio between the cardinalities may be close to 1 - e.g., between 0.9 and 1.1, or 1). The subsets corresponding to the outlier atoms may be referred to outlier subsets.

That is, the balanced set of medical images may admit a partition of the set of medical images into subsets. Moreover, a partitioning of the range of the meta-property may be given such that, for every subset of medical images of the partition, there is an atom of the partitioning of the range of the meta-property such that, for every medical image in the subset of medical images, the value of the meta-property of the medical image is an element of the atom. Furthermore, each atom of the partitioning may have a same size. A size may be a cardinality (e.g., if the range of the meta-property is finite) or a length (e.g., if the range of the meta-property is a real number). Whenever the range of the meta-property is finite, each atom of the partitioning may consist of only one element (so that the partitioning corresponds to the range itself) or more than one element. In these cases, each atom may contain the same number of elements (e.g., all atoms containing one, two, three, or more elements of the range). Whenever the range of the partitioning is an interval (e.g., the interval [0,1], or any other interval of the real numbers), each atom of the partitioning may be a sub-interval. That is, in these cases, the partitioning may consist in partitioning the interval in one or more intervals of the same length (e.g., partitioning the interval [0,1] into intervals [0,0.1], ..., [0.9,1], or into finer intervals).

The outlier subsets may have substantially less elements than the other subsets. The outlier atoms may correspond to outlier/extremal ranges of values of the meta-property. For example, if the range of the meta-property is the interval [0,1] and the partitioning is given by the intervals [0,0.1], ..., [0.9,1], the outlier atoms may be [0,0.1] and/or [0.9,1].

Therefore, a balanced set of medical images may be characterized by a partitioning of the range of the meta-property in atoms of a same size, and a corresponding induced partition of the set of medical images in subsets of a same cardinality.

The number of subsets of medical images of the partition and the number of atoms of the partitioning of the range of the meta-property may be the same. Therefore, there may be a one-to-one correspondence between the partition and the partitioning, so that each subset of the medical images may be uniquely associated with an atom of the partition of the range of the meta-property, the meta-property of each medical image of a subset being in the respective atom of the partition of the range. Alternatively, the number of subsets of medical images of the partition may be lower than the number of atoms of the partitioning of the range of the meta-property. For example, some atoms of the partitioning of the range may not correspond to any medical image (e.g., the range of the meta-property may comprise values that are not attained by any medical image).

Implementations of the dataset-forming method may comprise partitioning *M* = *M*₁ U ... U *Mₖ* the range *M* of the meta-property 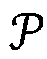 : *S* → *M*, where *S* is the third set of medical images. The partitioning satisfying:
- all the *Mᵢ* have a same size (e.g., a same cardinality, a same length); and
- the map 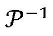 induces a partition *S* = *S*₁ U ... ∪ *Sₖ* (from the above-defined partition of *M),* such that all of the cardinalities |*S*₂|, ..., |*S*_{*k*-1}| are substantially the same (e.g., $0.9 \leq \frac{\left|{s}_{i}\right|}{\left|{s}_{j}\right|} \leq 1.1$ for all 2 ≤ *i,j ≤ k -* 1), and the cardinalities |*S*₁|, |*Sₖ*| ≤ |*Sᵢ*| for every 2 ≤ *i* ≤ *k* - 1.

It is understood that in the above implementations, the number of outliers may be smaller or larger (e.g., no outliers or *S*₁, *S*₂, *S*_{*k*-1}, *Sₖ* being outliers).

Therefore, a balanced set of medical images may be a set well representing the meta-property (e.g., having substantially the same number of images for each value or range of values of the meta-property). Using such a balanced set of medical images to train (e.g., machine-learning) a model may be beneficial as the model may be trained to be completely unbiased with respect to the meta-property (by having been trained on substantially the same number of images for each value of the meta-property). Indeed, using an unbalanced set of medical-images may bias the model to mis-predict the images corresponding to rare values of the meta-property.

The third set of medical images may be partitioned into subsets of medical images such that at least one of the subsets (e.g., a subset that is not an outlier) contains exclusively annotated medical images. For example, at least one of the subsets (e.g., a subset that is not an outlier) may contain exclusively annotated medical images of the first subset. This may be beneficial as this minimizes the amount of unannotated medical images in the third subset, while using all the annotated images (as the third subset comprises the first subset).

Implementations of the dataset-forming method may comprise obtaining and/or determining a partition *M* = *M*₁ U ... U *Mₖ* of the range *M* of the meta-property 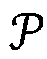 : *S* → *M.* The range *M* may be a finite set and each of the atoms of the partition *Mᵢ* may consists of a single element. Alternatively, the range *M* may be an interval and each atom of the partition *Mᵢ* may be a sub-interval of equal length, e.g., *M* = [0,1] and *M*₁ = [0,0.1], ... , *Mₖ* = [0.9,1]. The first, the second, and the third set of medical images may be denoted by *S*¹, *S*², and *S*³ respectively. The first and the second set are obtained (at S10 and S20 respectively). The map 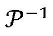 and the partition *M* = *M*₁ U ... U *Mₖ* induce a partition of the first and second set of medical images, ${S}^{l} = {S}_{1}^{l} ∪ … ∪ {S}_{k}^{l}$ for *l* = 1, 2. As *S*¹ is unbalanced, the subsets ${\left({S}_{i}^{1}\right)}_{i}$ do not have the same cardinality and there is an index *j* such that the subset ${S}_{j}^{1}$ has a maximum cardinality. For every *i* ≠ *j,* such implementations of the dataset-forming method may comprise computing the difference of cardinality ${d}_{i} = \left|{S}_{j}^{1}\right| - \left|{S}_{i}^{1}\right|$, which is by assumption positive, and may further comprise defining a set *Tᵢ* of medical images, by selecting a subset *Tᵢ* of ${S}_{i}^{2}$ of cardinality *dᵢ* if $\left|{S}_{i}^{2}\right| \geq {d}_{i}$, and setting ${T}_{i} = {S}_{i}^{2}$ otherwise. The subset *Tᵢ* may be selected in any way known in the art, e.g., considering random elements of ${S}_{i}^{2}$ or considering the first *dᵢ* elements with respect to a pre-established order. Such implementations of the dataset-forming method may further comprise setting *S*³ = *S*¹ ∪ (∪_{*i*≠}*ⱼ Tᵢ*). The map 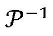 and the partition *M* = *M*₁ U ... U *Mₖ* induce a partition of the third set ${S}^{3} = {S}_{1}^{3} ∪ … ∪ {S}_{k}^{3}$ such that ${S}_{j}^{3} = {S}_{j}^{1}$ and ${S}_{i}^{3} = {S}_{i}^{1} ∪ {T}_{i}$ for every *i* ≠ *j*. Therefore, the so-defined third set of medical images is balanced, as $\left|{S}_{i}^{3}\right| = \left|{S}_{j}^{3}\right|$ for every *i* ≠ *j* such that |*Tᵢ*| = *dᵢ* (the other subsets may be outliers). As already mentioned above, the second set of medical images may be much larger than the first set of medical images, ensuring that the number of outlier subsets is small.

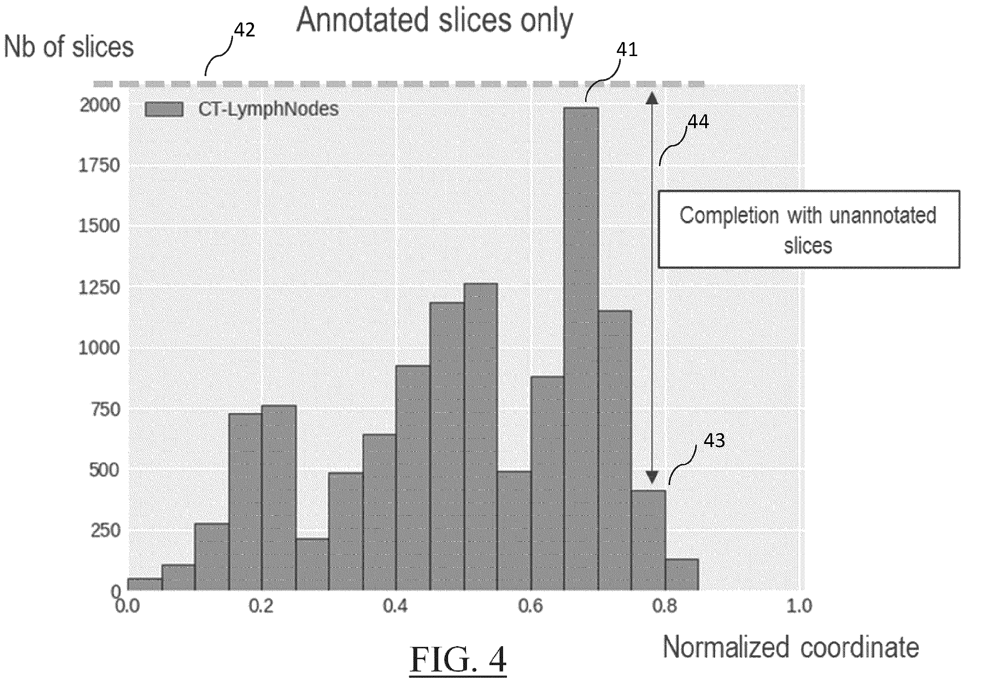

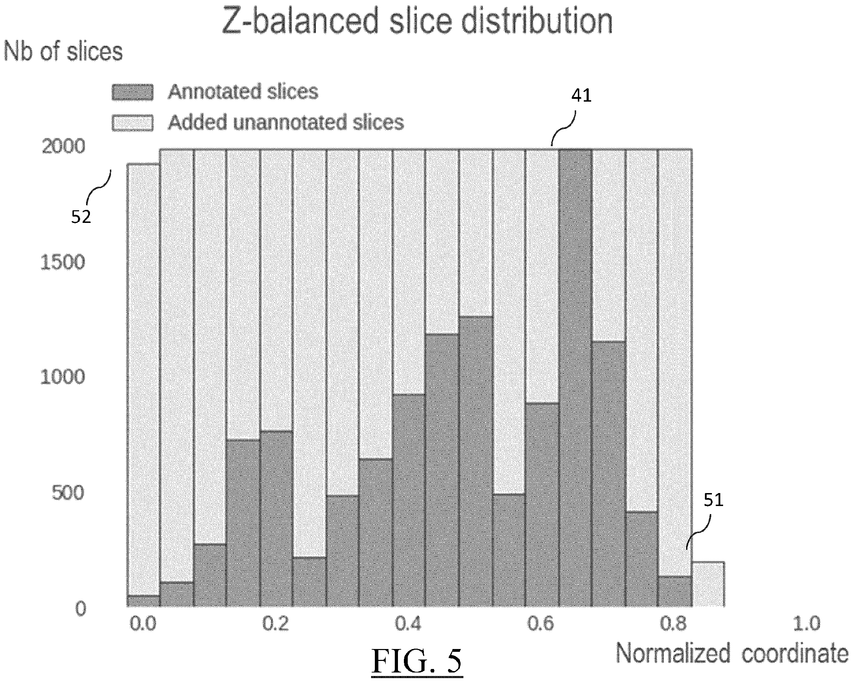

FIGs. 4 and 5 illustrate an example of the above-described implementation. As for FIGs. 2 and 3, the horizontal axis of the vertical bar charts of FIGs. 4 and 5 indicate the meta-property to be balanced and the vertical axis indicate a cardinality of images for the given meta-property range in the respective dataset. FIG. 4 illustrates the same vertical bar chart of the first set of medical images illustrated in FIG. 2. In FIG. 4, the subset of maximum size (denoted by ${S}_{j}^{1}$ in the above implementation) corresponds to bar chart 41. Its cardinality is taken as maximal cardinality for the subsets of the third set of medical images. This is illustrated with the dashed line 42. Given a subset, such as subset 43, the difference in cardinality is computed as illustrated in the double arrow 44. FIG. 5 illustrates the third set of medical images obtained by adding to the first set of medical images illustrated in FIG. 2 and 4, medical images of the second set of medical images illustrated in FIG. 3. As can be seen in FIG. 5, all subsets have the same cardinality, besides subsets 51 and 52. Subset 52 has substantially the same cardinality as the others, whereas subset 51 is an outlier. This is due to the fact that the second subset of medical images does not have enough medical images corresponding to the meta-property range of subset 51. This can be seen in FIG. 3.

At least one (e.g., each) of the medical images (e.g., of the first and/or second subset, or the input medical image) may be a slice image. As already mentioned above, a slice image may comprise an illustration of an internal anatomy of a patient and may be obtained, e.g., with a CT scan. The meta-property may comprise (e.g., consists of) a representation of a normalized height of the slice image. The slice image may be cross-sectional/transverse to the height of the patient and, therefore, may have an associated height, such associated height may be normalized in any way known in the art. A normalized height may be any real number (e.g., comprised between 0 and 1) expressing a distance from a reference point (e.g., the slice corresponding to height 0). The reference point may be the feet (e.g., just under the feet), or may be any other part of the body, such as the knees, thorax, or the pelvis. The normalized height may be obtained linearly (or using an affine function) from the usual height of a patient. In examples, the normalized height of a slice may be obtained by considering two reference points in the body, a lower reference point and an upper reference point, the lower reference point being associated with a normalized height of 0 and the upper reference point being associated with a normalized height of 1, all the points in between being associated with a normalized height between 0 and 1, linearly interpolating the values. The normalized height may be defined in such a way medical images representing same organs of different patients are assigned substantially the same normalized height (e.g., independent from the height of said patients).

A normalized height of a slice may correlate with the prediction of the model. For example, the internal anatomy of a patient highly depends on the height of the slice. In examples, the model may be trained to recognize a lesion, such as a cancer. As some cancers are more common than others (e.g., lung cancer is more common than other kind of cancer), the first set of medical images may be highly unbalanced (there may be a great difference between the cardinality of subsets of medical images corresponding to different ranges of the meta-property, as shown in FIG. 2). Indeed, there may be many more images of the thorax (e.g., segmenting lung cancers) than images of other parts of the body. Thus, the machine-learning method may enable training a model based on the third set that is able to correctly identify the presence of a cancer, even when said cancer is in a location of the body that is underrepresented in the first set of medical images. Moreover, as the normalized height of slices of different patients representing a same organ may be similar (e.g., exhibiting the same or very minor differences), the normalized height may allow comparing slices of different patients and training a model to accurately identify, classify, and segment anatomical features/diseases/lesions. This normalization may serve as a standard reference that aligns corresponding anatomical structures across patient datasets, reducing variability caused by differences in patient size or imaging protocols. Consequently, considering a normalized height may be beneficial for training a model.

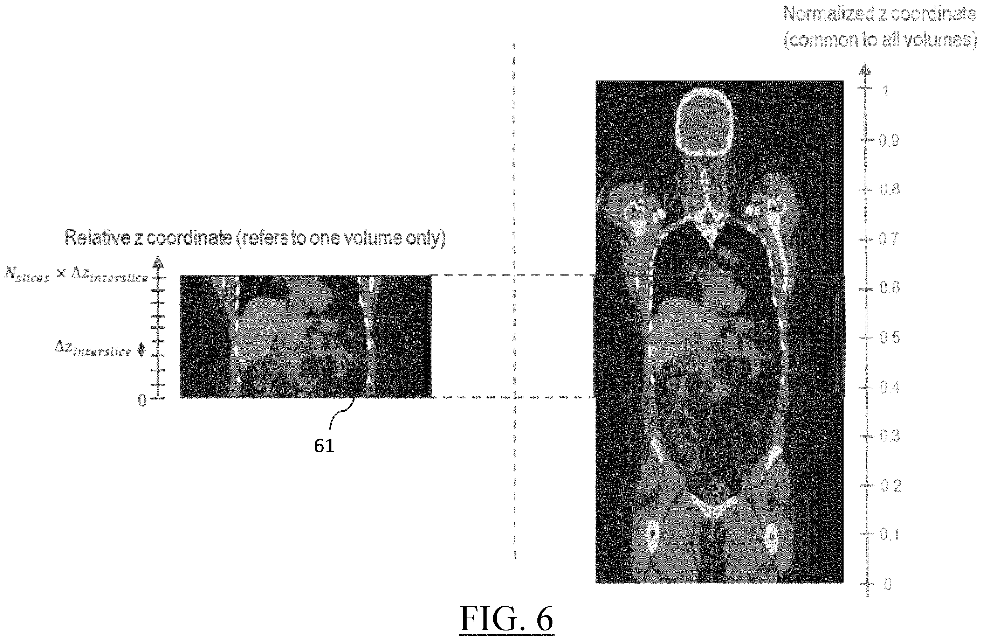

FIG. 6 shows an example of a coronal image of a patient comprising a normalized height coordinate scale, going from the legs to the head of the patient.

The images of the first set and/or of the second set of medical images (obtained at S10 and S20 respectively) may not comprise a ground-truth of a height (e.g., a ground truth of a normalized height) of the patient. In examples, this may happen since a slice, e.g., obtained with a CT scan, may not comprise the information of the height relative to the patient but only a relative height with respect to a body part of the patients (e.g., a first slice of the CT scan). This phenomenon is for example depicted in FIG. 6, where a smaller part of the body 61 is considered for a CT scan, and the height is taken relative to a first slice of the CT scan. As is known in the art, each CT scan slice is an image that refers to an anatomical location in a human body, ordered vertically. In the CT scan file metadata, the axial coordinates corresponding to each slice may be provided, however, this axial coordinate may be relative and proper to one CT scan only.

The representation of the normalized height (e.g., comprised in the meta-property) may be a result of a pretrained model configured to take a medical image as input, and to predict a value for the normalized height. For example, the dataset-forming method may comprise obtaining (e.g., inferring) a representation of the normalized height of at least one (e.g., each) of the medical images of the first and/or second set of medical images. The obtaining may be a result of a pretrained ZCI-model (Z coordinate inference model) configured to take a medical image as input, and to predict a value for the normalized height. That is, the meta-property may be a normalized height that is obtained using a pretrained ZCI-model. Therefore, the dataset-forming method may comprise:
- obtaining a pretrained ZCI-model configured to take a medical image as input, and to predict a value for the normalized height; and
- applying the pretrained ZCI-model to each of the medical images (of the first and/or second set of medical images), thereby obtaining the values of the meta-property for the medical images.

The obtaining of a pretrained ZCI-model may comprise retrieving (e.g., from local or remote memory) or receiving (e.g., from a remote system) the pretrained model. The pretrained ZCI-model may be implemented in different ways and may comprise different modules. The pretrained ZCI-model may be based on a convolutional neural network. In examples, the pretrained ZCI-model may be based on the work of Yan, K., Lu, L., & Summers, R. M. (2018, April) "Unsupervised body part regression via spatially self-ordering convolutional neural networks" in 2018 IEEE 15th International Symposium on Biomedical Imaging, pp. 1022-1025, that is included herein by reference. In implementations, the ZCI-model may comprise some of the modules of the above-referenced work, and may differ on other modules (e.g., comprise additional modules). The output of the pretrained ZCI-model may depend on the training dataset used for training it. For example, the pretrained ZCI-model may always (not depending on the training dataset) output a height between 0 and 1. However, the slice in the body corresponding to "0" or "1" may depend on the training dataset. In examples, when the pretrained ZCI-model may be trained on a dataset comprising slices form the pelvis to the neck, and the slice corresponding to "0" may be a slice representing a pelvis, the slice corresponding to "1" may be a slice representing a neck. A pretrained ZCI-model may additionally comprise a linear regressor to obtain a monotone, linear relation between the normalized height (e.g., when applied to an entire CT scan).

The obtaining of a pretrained ZCI-model may comprise:
- obtaining a ZCI-model to be trained;
- obtaining a training dataset of stacks (or equivalently volumes) of medical images, each medical image of each stack representing a slice image of a respective patient; and
- training the ZCI-model on the obtained training dataset, thereby obtaining the pretrained ZCI-model.

The training of the ZCI-model may follow the paradigm of unsupervised training (due to the fact that not enough slices with a ground truth of normalized height may be available). The training may comprise learning a relation order (corresponding to the order given by the stacks) between the slices. The training may be effectuated with random batching with varying learning rates and slice-distance. Each batch may consist of taking a number of stacks (e.g., CT scans of patients), e.g., 16 stacks, each stack may be separated into a number of equidistant slices, e.g., 8 slices. The first slice of each stack may be chosen randomly (e.g., among a set of slices at the beginning of the stack). In examples, the distance between the equidistant slices may decrease during training, so that the ZCI-module better learns to predict the normalized height.

In examples, the ZCI-module may comprise a convolutional neural network pretrained on other kind of images (e.g., non-medical), so that the training of the ZCI-module may require less images. In examples, one or more loss functions may be used and may have been adapted to the ZCI-module. In examples, a loss function may be used that heavily penalizes going against monotonicity.

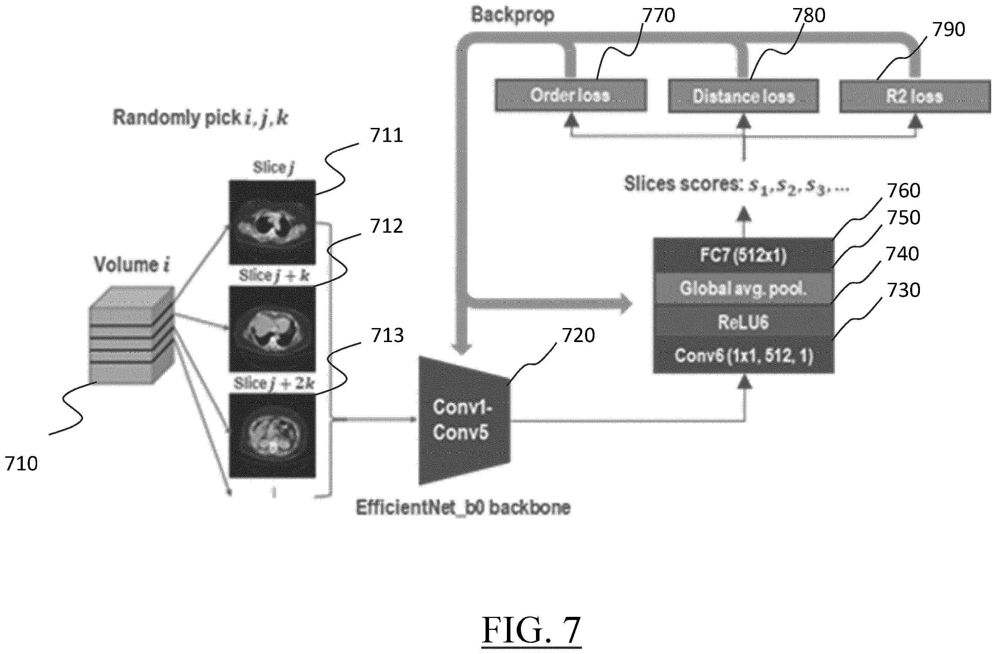

FIG. 7 illustrates an implementation of the ZCI-model and its training. In the implementation the training may be performed by:
- obtaining a training dataset of stacks of slices; each of the stack of slices may be a CT scan, e.g., representing a desired anatomical coverage of the human body tissue;
- uniformly choosing a stack i 710, a starting slice *j* 711 (from the stack i), and a step between slices k;
- sampling the chosen stack i by selecting a slice each k slices (e.g., the slice with index *j* + *k* 712 and the stack *j* + *2k* 713), starting with the slice with index *j* 711, until arriving to the desired number of slices in each stack 710, denoted as *n_slices_per_sample*; and
- feeding the sampled slices to the ZCI-model to be trained to regress slice scores one for each slice.

In the implementation, the ZCI-model may comprise a backbone responsible for extracting semantically relevant features maps, and additional head layers allow the regression of slice scores. The slices 711-713 are input to the ZCI-model via convolutional blocks 720. The neural network may also comprise layers Conv6(1x1,512,1) 730, ReLU6 740, a Global averaging pool 750 and FC7(512x1) 760. The layers output slice scores which are minimized with a loss, as explained below.

Preferably, the scores are regularly spaced, and correctly ordered as slices are also sampled regularly.

Regressing the slice score may comprise minimizing a loss. The loss may reward, for each slice score of a respective slice of the stack, at least one of an ordering of each slice score (e.g., denoted as an order loss term 770), attributing a regular spacing between pairs of slice scores (e.g., denoted as an distance loss term 780), and/or resembling to straight lines (e.g., denoted as an R2 loss term 790).

By rewarding an ordering of each slice score, it is meant that the loss penalizes the neural network for not ordering slices correctly. In other words, the loss penalizes that a higher slice (in the order set by the stack of slices) received a lower score than a lower slice. By rewarding a regular spacing between pairs of slice scores, it is meant that the loss penalizes the network for not attributing regularly spaced slice scores, that is, for two consecutive slices, the difference in slice score should be uniform. By rewarding resembling to straight lines, it is meant that the neural network penalizes slice scores output by the neural network that do not fit a straight line.

The trained ZCI-model thus outputs a common axial scale of normalized coordinates (in the form of the set of coordinates) for which a specific range of coordinates always refer to the same anatomical area. That is, a ZCI-model trained as in the implementation outputs a normalized height.

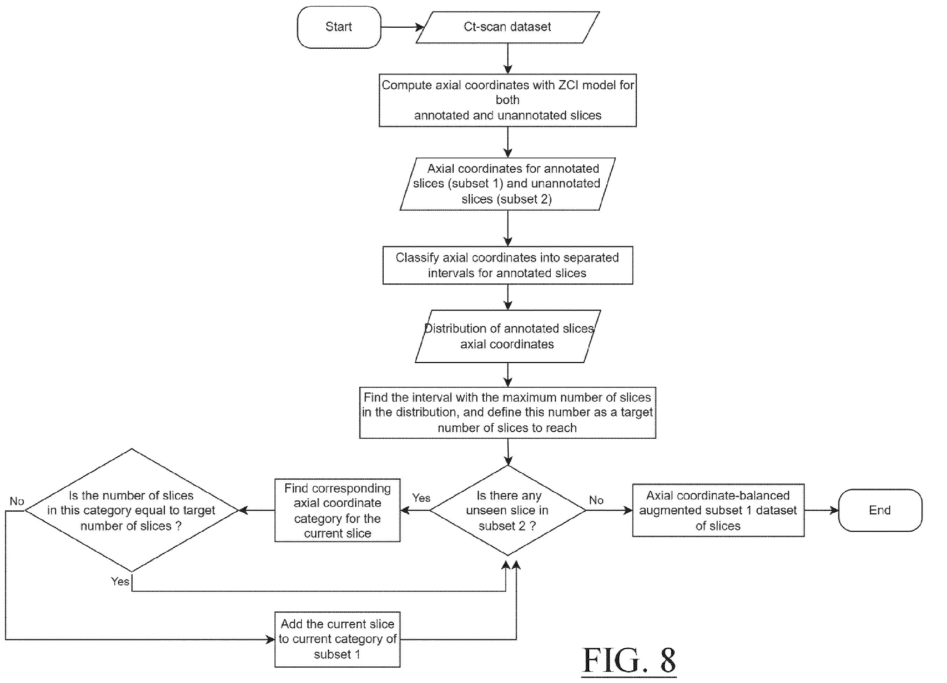

An implementation of the methods of the present disclosure is discussed herein. A flowchart of some of the steps of the implementation is illustrated in FIG. 8. In the implementation, the medical images of the first and second set are slice images, e.g., obtained with CT scans. In the implementation, the meta-property is a normalized height that is obtained with a pretrained ZCI-model.

In the implementation, each of the annotations is related to a lesion, e.g., to a tumor. In the implementation, each of the annotations may be a segmentation mask of a lesion or may be the information of the presence of the lesion (without the segmentation). In the implementation, the third set of medical images to be outputted is configured to be used for training a model. The model may be configured to output a segmentation of a lesion from a medical image provided as input.

The implementation comprises obtaining a dataset of slice images (e.g., of CT scans). The implementation comprises defining (corresponding to S10) the first set of medical images consisting of all the annotated slices of the obtained dataset. The implementation comprises defining (corresponding to S20) the second set of medical images consisting of all the unannotated slices of the obtained dataset.

The implementation may comprise obtaining a pretrained ZCI-model or training a model, thereby obtaining a pretrained ZCI-model. The ZCI-model and its training may be according to the above implementation. The implementation comprises providing as input each of the medical images of the first and second set to the pretrained ZCI-model to obtain a normalized height for each slice.

The implementation further comprises partitioning the range of the normalized height M = *M*₁ U ... U *Mₖ*. The range may be, e.g., *M =* [0,1] and the partitioning

may be, e.g., *M*₁ = [0,0.1], ... , *Mₖ* = [0.9,1]. The first and last interval, *M*₁ and *Mₖ*, of the partitioning may be considered outliers.

The implementation further comprises inducing the partitioning to a partition into subsets of the first set of medical images ${S}^{1} = {S}_{1}^{1} ∪ … ∪ {S}_{k}^{1}$ and the second subset of medical images ${S}^{2} = {S}_{1}^{2} ∪ … ∪ {S}_{k}^{2}$. The inducing may be implemented by using the inverse map of the meta-property 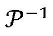 .

The implementation further comprises determining a maximum of the cardinalities of the subsets of the induced partition of the first set of medical images $C = {max}_{i} \left|{S}_{i}^{1}\right|$. The implementation further comprises, for each index of the partitioning 1 ≤ *i ≤* k, determining a subset of a respective subset of the partition of the second set of medical images ${T}_{i} ⊆ {S}_{i}^{2}$ having as cardinality |*Tᵢ*| *=* $min \left\{\left|{S}_{i}^{2}\right|,C-\left|{S}_{i}^{1}\right|\right\}$. It is noted that it might happen that ${T}_{i} = {S}_{i}^{2}$ if $\left|{S}_{i}^{2}\right| \leq C - \left|{S}_{i}^{1}\right|$ for some i, and that *Tᵢ =* Ø for other *i* (e.g., corresponding to a maximum cardinality subset). The determining of a subset of a respective subset of the partition of the second set of medical images may be implemented in any possible way, e.g., randomly, or following a predetermined order.

The implementation further comprises defining, and outputting (corresponding to S30), the third set of medical images as follows: *S*³ = *S*² U (∪ᵢ *Tᵢ*).

The implementation may further comprise training (corresponding to S40) the model based on the third set of medical images *S*³. The implementation may also further comprise obtaining an input medical image (e.g., provided by an health specialist) and applying the trained model to the obtained input medical image, thereby obtaining a prediction (e.g., whether the medical image contains a representation of a lesion, and optionally, a segmentation of said lesion).

The methods and implementations presented in the present disclosure may be combined in any possible ways unless explicitly stated otherwise. In particular, any of the methods described herein may comprise any of the steps described in relation to any of the described methods, implementations, and/or examples of implementations of the present disclosure.

The above implementation of the methods of the present disclosure was implemented in different settings (herein referred to as first, second, and third implementation). The results of such implementations show the efficacy of the method and are described herein below.

The results of a first implementation are described herein. In the first implementation two models based on the same architecture, with the same set of hyper-parameters are trained. The architecture is based on a framework Mask R-CNN and comprises:
- a backbone responsible for the extraction of the semantic features of the images,
- a Region Proposal Network (RPN) comprising a projection head responsible for the regression of bounding box coordinates in the image, and a regression head for the object presence score for each bounding boxes,
- a segmentation head for determining which pixels in the image correspond to the detected object, the segmentation head taking as input each of the objects of which score is higher than a predetermined threshold, and
- a classification head, in parallel to the segmentation head, responsible for tagging each lesion with an organ-tag (that is, the organ on which the lesion is located).

Both are trained using samples from a mix of CT-scan datasets (CT-LymphNodes, MSD-HepaticVessel, UniToChest). The first model is trained based on the mix without balancing (that is, based on the first set of medical images, without using the dataset-forming method). The second model is trained on the mix after balancing (that is, based on the third set of medical images, after using the dataset-forming method). The validation dataset (CT-ORG) used for testing the models is the same, thus enabling fair comparison of the performances. The evaluation is performed using the AP50 metric (Average Precision at loU *=* 50%). The AP50 metric is a common evaluation metric in object detection tasks and measures the area under the Precision-Recall (PR) curve. It summarizes the model's ability to make accurate predictions (precision) while capturing all relevant objects (recall), under a constraint of 50% loU with a ground-truth to consider a bounding box a "true positive". The results of the first implementation are as follows:

| | AP50↑ |
|---|---|
| Model 1 (unbalanced) | 0.2205 |
| Model 2 (balanced) | 0.2691 |

Therefore, it may be appreciated that the dataset-forming method greatly increase the performance of the model, increasing the AP50 by almost 0.05, which is a noticeable improvement. Moreover, this result still holds for different starting training dataset. That is, the increase performance does not depend on the specific dataset used in the first implementation.

The results of a second implementation are described herein. The second implementation is similar to the first implementation: two models based on the same architecture, with the same set of hyper-parameters are trained. Both are trained using samples from a same dataset (DeepLesion dataset). The first model is trained based on a dataset without balancing (that is, based on the first set of medical images, without using the dataset-forming method). The second model is trained on a dataset after balancing (that is, based on the third set of medical images, after using the dataset-forming method). The validation dataset used for testing the models is the same, thus enabling fair comparison of the performances. The two models are based on a truncated model (comprising some of the modules of the first implementation), the truncated model only outputting bounding boxes. Specifically, the two models are composed of a backbone that extracts semantic features from images and are then followed by a RPN (Region Proposal Network) architecture, which is responsible for proposing region of interests where target objects are likely to appear. As the models only predict bounding boxes, 2D validation metric curves are provided as evidence of the effectiveness of the dataset-forming method.

FIG. 9 shows the two curves, the curve 91 corresponds to the first model (trained only on the first set) and the curve 92 corresponds to the second model (trained on the third set). As can be seen, the AP50 is increased by approximately 0.05 using the dataset-forming method.

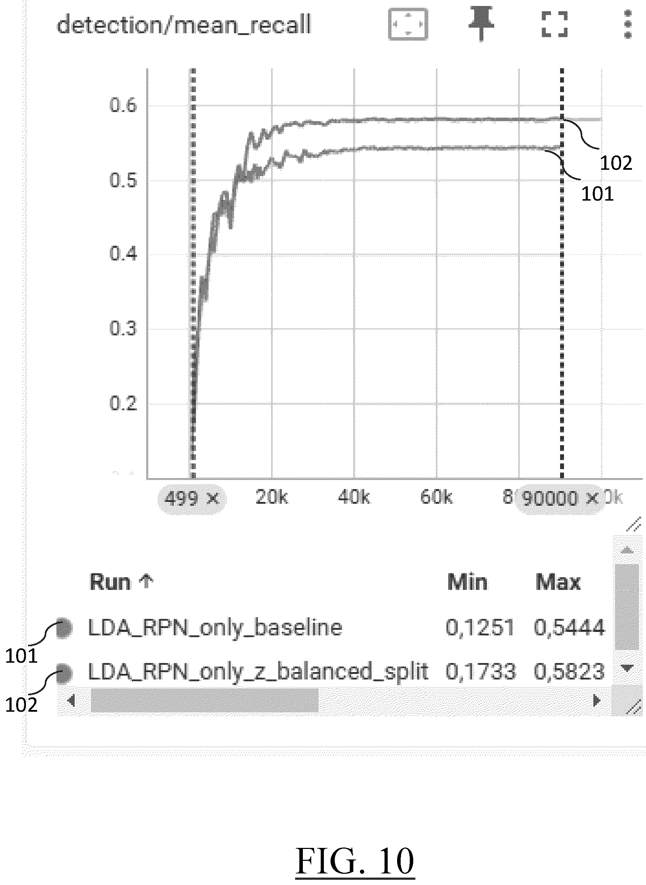

FIG. 10 shows the two curves, the curve 101 corresponds to the first model (trained only on the first set) and the curve 102 corresponds to the second model (trained on the third set). As can be seen, the mean recall (which is the average of recall metrics for different rates of false positives) is increased by approximately 0.04 using the dataset-forming method.

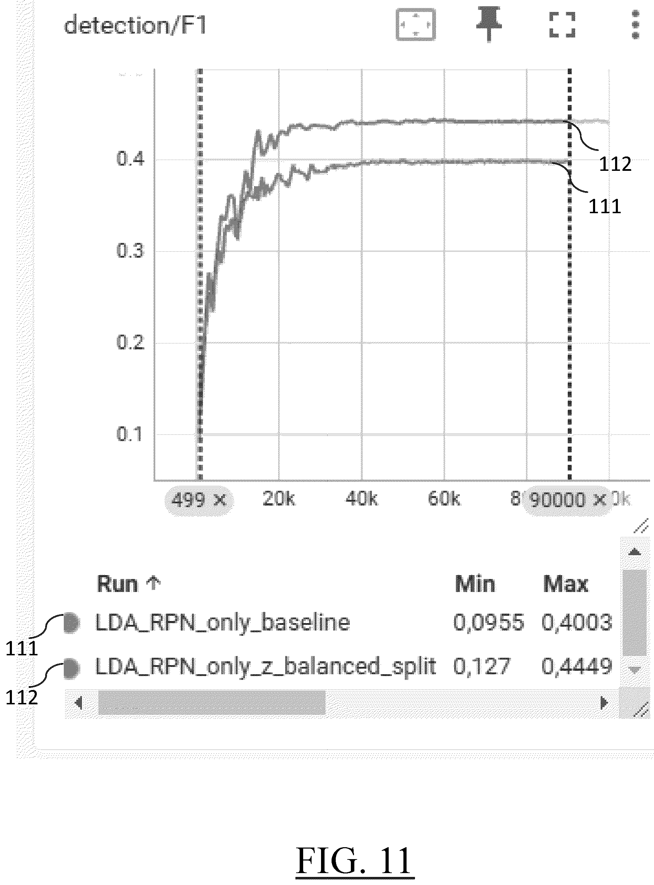

FIG. 11 shows the two curves, the curve 111 corresponds to the first model (trained only on the first set) and the curve 112 corresponds to the second model (trained on the third set). As can be seen, the F1-score (which is the harmonic mean of recall and precision) is increased by approximately 0.04 using the dataset-forming method.

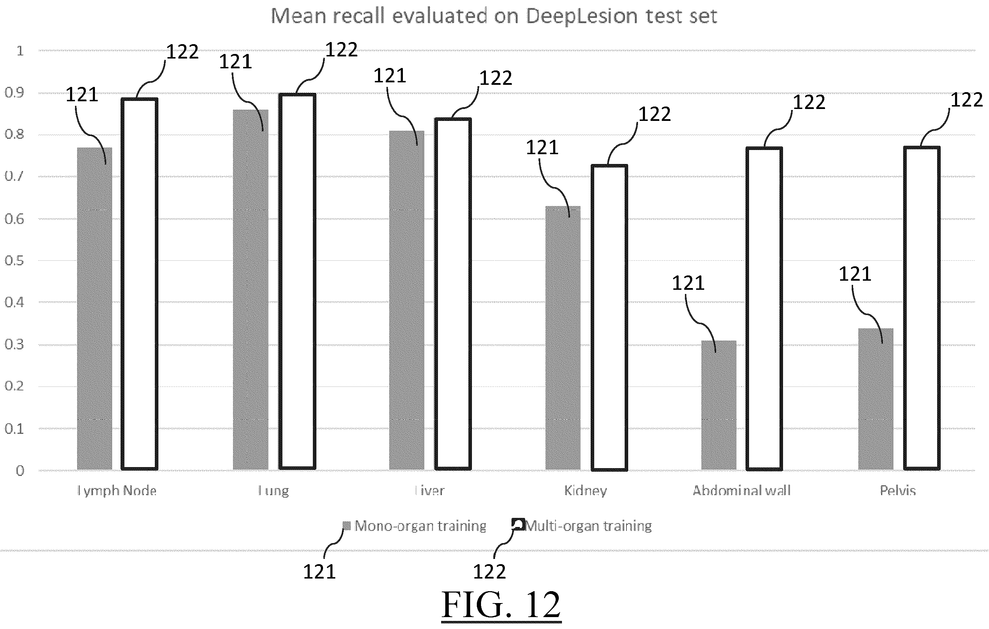

The results of a third implementation are described herein. In the third implementation, several models based on the same architecture are trained. All the models are trained based on samples from a same dataset (DeepLesion dataset) which presents a mix of cancerous lesions on different organs in the human body. The dataset is split into training/validation/test sets, which represents 80%, 10%, and 10% of the dataset respectively. In this dataset, organ tags are given along with lesion annotations to know on which organ a lesion lies. The first model (herein multi-organ model) of the several models is trained on the whole training set. The model is thus trained for performing the detection task on each of the organs. The other models (herein mono-organ models) of the several models are trained each on a respective restriction of the dataset. Each of the mono-organ models is trained only based on the images comprising lesion annotations corresponding to a respective organ (lymph node, lung, liver, kidney, abdominal wall, and pelvis). The multi-organ model was evaluated on the whole test set. Each of the mono-organ models is evaluated on a restricted test set consisting of the slice images representing lesions belonging to a respective organ. The results are shown in FIG. 12.

FIG. 12 shows a vertical bar chart, where the vertical bars 121 show the results of the mono-organ models and the vertical bars 122 show the results of the multi-organ model. The multi-organ model has better performances than each of the mono-organs models. This means that the multi-organ model learns a more robust lesion representation than its specified counterparts. In conclusion, the concept of lesion learnt by the multi-organ model benefits from the lesion diversity it encounters during training.

The methods of the present disclosure are computer-implemented. This means that steps (or substantially all the steps) of the methods are executed by at least one computer, or any system alike. Thus, steps of the methods are performed by the computer, possibly fully automatically, or semi-automatically. In examples, the triggering of at least some of the steps of the methods may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

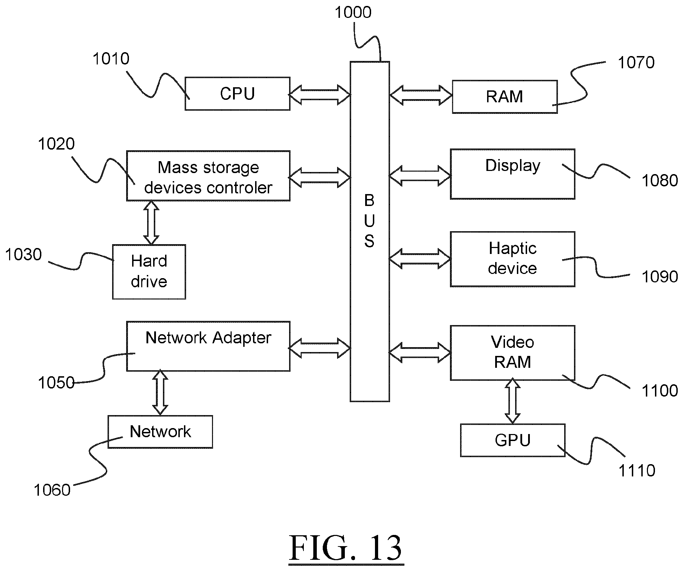

FIG. 13 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer-implemented method for forming a dataset of medical images, the dataset being configured to be used for training a model, the trained model being thereby configured to output a prediction from an input medical image, the dataset-forming method comprising:
• obtaining (S10) a first set of medical images, the first set consisting of annotated medical images, the first set being unbalanced with respect to a meta-property relative to each medical image;
• obtaining (S20) a second set of medical images, the second set comprising unannotated medical images; and
• outputting (S30) a third set of medical images, the third set comprising the first set and unannotated medical images of the second set, the third set being balanced with respect to the meta-property.

2. The method of claim 1, wherein the prediction comprises a segmentation of the input medical image.

3. The method of claim 2, wherein each annotated medical image of the first set has at least one annotation that represents a lesion, and the segmentation comprises a lesion segmentation, the lesion optionally being a tumor.

4. The method of claim 3, wherein the unannotated medical images of the second set included in the third set comprise medical images where a lesion is represented.

5. The method of any one of claims 1 to 4, wherein each medical image is a slice image of a respective patient, optionally a slice of a Computed Tomography scan (CT scan) of the respective patient.

6. The method of claim 5, wherein the meta-property comprises a representation of a normalized height of the slice image.

7. The method of claim 6, wherein the representation of the normalized height is a result of a pretrained model configured to take a medical image as input, and to predict a value for the normalized height.

8. The method of any one of claims 1 to 7, wherein the third set of medical images is partitioned into subsets of medical images, each subset corresponding to a respective atom of a partitioning of the range of the meta-property, each atom of the partitioning having a same size.

9. The method of claim 8, wherein at least one of the subsets contains exclusively annotated medical images of the first subset.

10. A method of machine-learning a model, the model being thereby configured to output a prediction from an input medical image, the machine-learning method comprising:
• obtaining a dataset formed according to any one of claims 1 to 9, the dataset being the outputted third set; and
• training the model on the dataset.

11. A machine-learnt model obtainable according to the method of claim 10.

12. A computer-implemented method for using the machine-learnt model of claim 11, the method of use comprising:
• obtaining a medical image; and
• applying the machine-learnt model to the medical image.

13. A computer-program comprising instructions which, when the program is executed on a computer, cause the computer to carry out the method of any one of claims 1 to 8, the method of claim 10, and/or the method of claim 12.

14. A computer-readable storage medium having recorded thereon the computer-program of claim 13 and/or the machine-learnt model of claim 11.

15. A system comprising a processor coupled to a memory, the memory having recorded thereon the computer-program of claim 13 and/or the machine-learnt model of claim 11.
